(19) Europäisches Patentamt
European Patent Office
Office européen des brevets

(11) EP 1 935 244 A1

(12) **EUROPEAN PATENT APPLICATION**
published in accordance with Art. 153(4) EPC

(43) Date of publication:
**25.06.2008 Bulletin 2008/26**

(21) Application number: **06821840.3**

(22) Date of filing: **11.10.2006**

(51) Int Cl.:
***A01K 67/027*** *(2006.01)* ***A61K 45/00*** *(2006.01)*
***A61P 25/28*** *(2006.01)* ***C12N 15/09*** *(2006.01)*
***G01N 33/15*** *(2006.01)* ***G01N 33/50*** *(2006.01)*

(86) International application number:
**PCT/JP2006/320343**

(87) International publication number:
**WO 2007/043589 (19.04.2007 Gazette 2007/16)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **12.10.2005 JP 2005298049**
**24.07.2006 JP 2006200234**

(71) Applicant: **Astellas Pharma Inc.**
**Tokyo 103-8411 (JP)**

(72) Inventors:
- **MATSUMOTO, Mitsuyuki,**
**Astellas Pharma Inc.**
**Tokyo 1038411 (JP)**
- **MATSUMOTO, Shun-ichiro,**
**Astellas Pharma Inc.**
**Tokyo 1038411 (JP)**

(74) Representative: **HOFFMANN EITLE**
**Patent- und Rechtsanwälte**
**Arabellastrasse 4**
**81925 München (DE)**

# (54) ANIMAL MODEL OF SCHIZOPHRENIA

(57) A non-human transgenic animal exhibiting schizophrenic symptoms in which a polynucleotide encoding SREB2 whose overexpression in the brain causes schizophrenia and a polynucleotide comprising a promoter are introduced; and a method of screening for a therapeutic agent for schizophrenia, comprising the steps of administrating a test substance to the animal, determining a schizophrenia-related disorder in the animal, and selecting the substance exhibiting a therapeutic effect on schizophrenia are disclosed. According to the present invention, a model animal of schizophrenia in which the genetic background that causes schizophrenia is reflected, and an in vivo screening method for a therapeutic agent for schizophrenia can be provided.

EP 1 935 244 A1

SEQUENCE LISTING

<110> Astellas Pharma Inc.

<120> Model animal of schizophrenia

<130> A06042-768

<150> JP 2005-298049
<151> 2005-10-12

<150> JP 2006-200234
<151> 2006-07-24

<160> 12

<170> PatentIn version 3.1

<210> 1
<211> 12742
<212> DNA
<213> Artificial Sequence

<220>
<223> Description of Artificial Sequence: a sequence for preparing a transgenic mouse

<220>
<221> CDS
<222> (7985)..(9094)
<223>

<220>
<223> Inventor: Matsumoto, Mitsuyuki; Matsumoto, Shun-ichiro

<400> 1
gacgtcgatc ttttttccgt aaactcaata ccaggctgat gtcccaccgg atctgatggc 60

ttagggtggc agggaatctc agttcccctc agacactctc cctttgctgg ttctcaggga 120

ggaggcaagg tcaagtcttc atctgtaggc acgtggaggg agggcacaga agccctcagc 180

tgaatagggt gggacttggg gaagggcagc aaccaggctg ggttgcctgg gtcacaatcc 240

tgcctctttc ctgatgagtt tcctttttgc cctcaggtta cctatagcag cattctgcct 300

caatctcacc cctaagatga gctctggtga ctttaggact ccagtgtaca catgtgtctg 360

gggccatggc agggtttctt gctgaccttg tcaccttcca gacaacttga gtccatgacc 420

ctctttccag ctctctgtgg tgctcttgga tatcagctgg agtatggcca gctggctgct 480

gctctgttga acaactcaat gagagaacgg acagggtagg ctctgagaaa tctttacgtt 540

cctggagcct catgacttgg gagcctagtg gaattcttct cttttggtcc ccaacatctg 600

gggggagggg gaactggctg agcctgagcc actgtatagt gagggtgggg gaagcagctg 660

tgaaaggagc ctttgatttg gtcttgaaca cagttcttcc ccacagggcc ttgatttccc 720

tacttgcaaa ggagtaggga aatatgagcc ttggctctgc ctacctcacg ttgctggtgc 780

tgtagaaaac tggccaggct gatggttgtg gaggagcctg tgaacttgat taaagtgcca 840

ttatccagag gcaagagatg ctggtctgtg tgtgtttgag tgtgtgtgtg tgtgtgtgtg 900

tgtgtgtgtg tgtgtgtgtg tgtgtgtgtg tgtgtcttgg agacctgtgt gagctcgatg 960

ctagcagagg ggacagaagg taggtgggaa ggaaggaagg aatgaaggaa ggaaagaagg 1020

aaggttgaaa ggaagaaagg aaggaaggaa ggaaggaagg aaggaaggaa ggaaggaagg 1080

aaggtcctgc cacaggctta ccatgtagct gcaggcaaac ccctgaccct ctctgggcct 1140

aagtgtttct ctacacacaa tggatgattc aagagtcctt acttttggtg gttacaggca 1200

cccctgtgca catttgcatc tggggtgggg gggacacagg cttggtagtg ttgaggaggg 1260

gggtggttgt agagcctgct agctgcatag cgctgctctc tccgcacact gcgttctgca 1320

tatctccctt caggtcccag tcggcgcagt gtgtgtaggc gaaggccctg ctgtgaattt 1380

tgaaaatagt tatttttgtc actggcaaag gaggccctgt taggactcgt cagcttgtgg 1440

atgagcggga tgggtggagt ggggtgggtg cggtgccgcc gtgggggggtt accgctgctt 1500

gcagggctgc atcgcccagg cagtgactga atcctgcatg agggcctggc ctaggctgtg 1560

gggaggagat gaccactgcg tcctagatct ttccttagcc ctgtgcttcc tttcttcctt 1620

ttttccttaa gatttatttc taatgatgcg taggttgtgt atttgtgtgt gggtatgtgc 1680

acttgaataa aggacccaca gaggctatag acatcagatc ctcctagagc tggggttaca 1740

```
gagggcgtga gttgtccaac atgggcaccg gaaaataaac ttatgtgctc tacacgaccg 1800
agctgtctct ccaacaccag cccccttctt ttgacttttc tcatctccct cacttgtatg 1860
ttttcccttc ctcgataatg ctgataccca gatggtaggc acggcccagg ataggcaggg 1920
gtctctgcgc tccagtggct gtagtggttt tcctgcctgc tctgaagaag acactggcta 1980
aggtggtgtc ttagcctact gtatcctaga ggtgggttat tcatagtctg cccactgccc 2040
aacacactct aggcctgctg gggcctactc taactctgct tgctggcttg ccaccctggc 2100
acacagtgta ggcttccctg tagagccagg ctttagaaaa ctgtatgagt acttctctga 2160
gaactgctgg aggggccctg cctgccagga cttttcaact tccagccctg tcatctatat 2220
cacttctgag gaccccgtgt gggggtcacg agaacaagac catatgtagt gctttctgtt 2280
ctcccttggg tcccaggctc tgaatcaatc tggtcccaag atataaggga tgattggtgc 2340
tgaggctggt gtctgtttct gaagttttga agacaagggt tggctcaagc ctccctgtgt 2400
tcagtcctcc actcaatgca gaactcagtg aactcagaat tctcagccca gatgccagca 2460
tagcccagca tagcccagca tagcccagga gctactggag catcagtttg aaaccaggtc 2520
cgcaggaact agtgggcaac agtgtgtgag gccagtggtc tttggggtat tgtattgaat 2580
tgagaggtcc tgcttagcag tcagcatgcc cacaacctgt tctctacggt ggtccggatt 2640
cccctcagca agcacacctg aatctttact acatcccagt tcctggttgg ctcctgactt 2700
cgggttacta tggctgtgat gaaacactac gactaaaagc aatgtgggga agaaagttaa 2760
ttttttact cgaccttcca tagacagggt tcatcactaa aagcagaggg cagaggaaaa 2820
gatagctcag cggttaagag tgctgtctac tcaacaaaga ggtcttgagt tcaattccca 2880
gcaaccacat ggtggctcac aactgtctat cctgggatct gatgcccttt tctggcatac 2940
aggtatacat acagatagag gactcatata cataaaataa ataaataaat ctttaaaagc 3000
aacaagggca gaaattcaag cagggcaggg acccagaggc caaagctgac gcagaggcca 3060
tggaggggtg ttgcttactg gcttgctcct catggcttgc tcagcctgtt ttcttataga 3120
acccacaacc accaggccag agatgggacc acccacaaag ggcagagcct ttccctatca 3180
```

atcactaatg ggaaaacatc ctgcagtcgg atcttatgaa gacattttct cagctgagct 3240

tccctcctat cagataactc tagcttgtgt caaggtgact taaaactagc cagcacagca 3300

ccttatgctc acatcacctg ggtccctttg gagaggacat agttaaaggg agcccagagg 3360

cagtccctag gccacaggtc ttcattgccc ctctctggga cggattagac aggctgcaga 3420

cctgttagct ggaagagtta gattcaggca agagcttgaa tctttacctg atcctggcta 3480

tggagtcctg gcctctaatg atcagctccc taacaaccca atggagccat atacctgcct 3540

gggccatggc tgtgtctcct cttctttcag acactcctgg cttgcctagg acacaggcta 3600

gcatcctgtc aatgccagga aggggcacag cagggaaaga gcaatgctgt tggcctgact 3660

gccaccaact ggtgtacctg ttagagggca acctctattc tctgcacctt ggttcctagc 3720

tctaagggat atgtggcccc taaaggtctt catagcttga tatgggaggc agggggggcta 3780

agaacagcgc aagagtggtg agcttgcaca gacccggatt tgatctctgg gtgagtgagg 3840

aggaaatgag atgggggtgg gggaagccct atttctagct gtcttagcat aggaactgaa 3900

cctccttctg cagggcctgt gtcactgccc ctttccccca gggagggccc ctgcacgggg 3960

cacctcaggg cacagccctt tttccctccc tcctctctta gacctggaat tactcaacat 4020

cctgccctga ctcagttgct ctcccctcag accctcacag tcttccttct cttctggccc 4080

acttttggct gagcctgccc ccaacttttt ctgcccttag tgggacaggc cccatgggga 4140

ccattcagat ggcacttttt tcccccccctg gggtggtttt ctgtggtggt gccctattca 4200

ggcaactgca agaccctgtg gcatttagca tatgcatgag agcacatgaa gaagctagct 4260

atccctgtgt gctgaggatt gtaatcctct ctcatccttc ccttgtctcc tggaacccag 4320

cccagcctcc tgtccctccc gttgacacga gccaatgctg gctcagcaaa ctccagggct 4380

cccacccctg gccatcagcc cttggcacac aggcttgtgc ttgagtactg cacacgtgtt 4440

gcagctgggg tacacgtgct ggactgttat gcctactgtg gccccggggg tgtgtgggaa 4500

gtctggcaga accaatccct ccatcccccg atgcaatcat cagcttattc tctcacggcc 4560

actcgggcat gcttgactcc ttgatgcccg ccgccactag gcacagctgc cagctttgtg 4620
ggcacagagg atgtggcgaa ttagtggtca tgcctcctca gtggaatggc aattgcactc 4680
agcatgcagg tgtctaccaa aggcagtccc tacatccccg atgtactctc gagacccatc 4740
taaggactag atctagtctc tagaaggtcc catgcagatg taagacagcc ctccacaggg 4800
agattcttcc agctagttct ctattatcag atgggtctaa gatcctagga cctgcctatc 4860
ccttagccct gcattcagcg agagaagggg taaagatgtg aggatgccag ggaggaagga 4920
aaagggcaca aggaagaaag aaagggaagg aagctggaag catggaagga caaagatggt 4980
gaccacagta gaattaggat cccatggttc ctgtcagtgg caggcagcta tcaggctgtc 5040
tgtgcctccc tgagcccctg gggcatcttc taaatgcttt gctggcctct gagccaagca 5100
ctgcatacca tcccgtgggg agtgacaggc cagcactggt caacgaggat gatggctact 5160
tttgttcaca gggtaacatc tccatggtta cagcctttgc acattcctct tagtacttta 5220
ccaatctcaa agcagttgcc aagcccttgg gccctaataa gtgagggtcc cagtgccctc 5280
tttttaaaat tccttgccat ttgttttgca gaatttactg caaataaagc caaccccagg 5340
caatgtctaa accatgagtt aaccccccag caaggtctca gagaactgtg ccccagagag 5400
ctgccaaggt tcagggagga gtatgaggag acaggatttc tagttcctta ataattcctt 5460
ctgtctcagc cactgtgttc atcttgtttc agccacaaaa ctacctttat tggtaaggaa 5520
cattatttac ccagtttcac acttgaagag gtccagagac gttaacacat cgattcaaaa 5580
gcacagcctg taagtcacat agccactgtt agctgatcga cactatttcc cctgggcaat 5640
ggctgggtga ttccagggat ccccttggga acaggctaga gcactggctc tcaacctgtg 5700
cgggtcgtga cctccttgag gggtaggggt gagggcagtg tcaaacaacc cttttacagg 5760
agtcgtttaa gaccgttgga aaaaaaacca gatatttgca ttatttttcg taacagaagc 5820
aagattatag ttatggagta gtgacaaaaa ttatgttaca gttggaggtc agcacagcat 5880
gaggaactgt atttaagggt tgcggcatta ggaaggttga gaatcactgg cctagcggat 5940
ctgaatcagg aacacggacg tacagctctg cgccactcct gccttcctct ggtgcctcta 6000

gccttgccca tggtgttctg ggcctgcctg ctacccacca gctgtgcggc cctgtgagca 6060

caggcctttc tgctccgctc tgaattgcca cgttggcggc agaagcggga agcgtattgt 6120

gcacagaaac aaaacggagt ggtttttttt tccttttttct gaaggtggta atggtgcaat 6180

tagtggcgaa gccatcaccc cctcctcccc ggctcgcctc cctccttcct ctccacctcc 6240

cttctctttc tttcctgaga aaaaaagtgg ctgagttgaa aagatctccc gtcaatcttt 6300

ctgtaacgga ctcaggaaga gggatagagg gccccctaat gtttccaggg tcctcgagcc 6360

tcagttgggt caggcacttg ttggtgctgg agaatattca aaggtaccac tatgttcccc 6420

acaagggagt tgagcaatgg attctgagga gcaagtttga aacagagaat ttgcgttccc 6480

aggtcttgtg atctgcccct tgttcactgg gggacaaatg ctggcatgag accctgagac 6540

ctctgctcag ccacctttct ctctctctct ctctctcttt ctctctctct ctctctctct 6600

ctctgtcctt aatggaggtg tgtgtgtgtt caagaccaag ctgcagtgtt ggagtgcttg 6660

tgggctcatt ttaaaacttc catgttttgc cttctagaaa ctgaaacata agaaccccat 6720

tatggcctta ggtcacttca tctccatggg gttcttcttc tgattttcta gaaaatgaga 6780

tgggggtgca gagagcttcc tcagtgacct gcccagggtc acatcagaaa tgtcagagct 6840

agaacttgaa ctcagattac taatcttaaa ttccatgcct tgggggcatg caagtacgat 6900

atacagaagg agtgaactca ttagggcaga tgaccaatga gtttaggaaa gaagagtcca 6960

gggcagggta catctacacc acccgcccag ccctgggtga gtccagccac gttcacctca 7020

ttatagttgc ctctctccag tcctaccttg acgggaagca caagcagaaa ctgggacagg 7080

agccccagga gaccaaatct tcatggtccc tctgggagga tgggtgggga gagctgtggc 7140

agaggcctca ggaggggccc tgctgctcag tggtgacaga taggggtgag aaagcagaca 7200

gagtcattcc gtcagcattc tgggtctgtt tggtacttct tctcacgata aggtggcggt 7260

gtgatatgca caatggctaa aaagcaggga gagctggaaa gaaacaagga cagagacaga 7320

ggccaagtca accagaccaa ttcccagagg aagcaaagaa accattacag agactacaag 7380

ggggaaggga aggagagatg aattagcttc ccctgtaaac cttagaaccc agctgttgcc 7440

agggcaacgg ggcaatacct gtctcttcag aggagatgaa gttgccaggg taactacatc 7500

ctgtctttct caaggaccat cccagaatgt ggcacccact agccgttacc atagcaactg 7560

cctctttgcc ccacttaatc ccatcccgtc tgttaaaagg gccctatagt tggaggtggg 7620

ggaggtagga agagcgatga tcacttgtgg actaagtttg ttcgcatccc cttctccaac 7680

cccctcagta catcaccctg ggggaacagg gtccacttgc tcctgggccc acacagtcct 7740

gcagtattgt gtatataagg ccagggcaaa gaggagcagg ttttaaagtg aaaggcaggc 7800

aggtgttggg gaggcagtta ccggggcaac gggaacaggg cgtttcggag gtggttgcca 7860

tggggacctg gatgctgacg aaggctcgcg aggctgtgag cagccacagt gccctgctca 7920

gaagccccaa gctcgtcagt caagccggtt ctccgtttgc actcaggagc acgggcaggt 7980

```
cgac atg gcg aac tat agc cat gca gcc gac aac att ttg caa aat ctc     8029
     Met Ala Asn Tyr Ser His Ala Ala Asp Asn Ile Leu Gln Asn Leu
     1               5                   10                  15

tcg cct cta aca gcc ttt ctg aaa ctg act tcc ctg ggt ttc ata ata      8077
Ser Pro Leu Thr Ala Phe Leu Lys Leu Thr Ser Leu Gly Phe Ile Ile
                20                  25                  30

gga gtc agc gtt gtg ggc aac ctt ctg atc tcc att ttg cta gtg aaa      8125
Gly Val Ser Val Val Gly Asn Leu Leu Ile Ser Ile Leu Leu Val Lys
            35                  40                  45

gat aag acc ttg cat aga gct cct tac tac ttc ctg ctg gat ctg tgc      8173
Asp Lys Thr Leu His Arg Ala Pro Tyr Tyr Phe Leu Leu Asp Leu Cys
        50                  55                  60

tgc tca gac atc ctc aga tct gca att tgt ttt cca ttt gta ttc aac      8221
Cys Ser Asp Ile Leu Arg Ser Ala Ile Cys Phe Pro Phe Val Phe Asn
    65                  70                  75

tct gtc aaa aat ggc tcc acc tgg act tac ggg act ctg act tgc aaa      8269
Ser Val Lys Asn Gly Ser Thr Trp Thr Tyr Gly Thr Leu Thr Cys Lys
80                  85                  90                  95

gtg att gcc ttc ctg ggg gtt ttg tcc tgt ttc cac act gcc ttc atg      8317
Val Ile Ala Phe Leu Gly Val Leu Ser Cys Phe His Thr Ala Phe Met
                100                 105                 110
```

ctc ttc tgc atc agc gtc acc aga tac tta gcc atc gcc cat cac cgc 8365
Leu Phe Cys Ile Ser Val Thr Arg Tyr Leu Ala Ile Ala His His Arg
115 120 125

ttc tat aca aag agg ctg acc ttt tgg acg tgt ttg gct gtg atc tgc 8413
Phe Tyr Thr Lys Arg Leu Thr Phe Trp Thr Cys Leu Ala Val Ile Cys
130 135 140

atg gtg tgg act ctg tcc gtg gcc atg gca ttc ccc cca gtt tta gat 8461
Met Val Trp Thr Leu Ser Val Ala Met Ala Phe Pro Pro Val Leu Asp
145 150 155

gta ggc acc tac tcc ttc att agg gag gag gat caa tgc acc ttc caa 8509
Val Gly Thr Tyr Ser Phe Ile Arg Glu Glu Asp Gln Cys Thr Phe Gln
160 165 170 175

cac cgc tcc ttc agg gct aac gat tcc cta gga ttt atg ctg ctc ctt 8557
His Arg Ser Phe Arg Ala Asn Asp Ser Leu Gly Phe Met Leu Leu Leu
180 185 190

gct ctc atc ctc cta gcc aca cag ctt gtc tac ctc aag ctg ata ttt 8605
Ala Leu Ile Leu Leu Ala Thr Gln Leu Val Tyr Leu Lys Leu Ile Phe
195 200 205

ttt gtc cac gat cga agg aaa atg aag cca gtc cag ttt gta gca gca 8653
Phe Val His Asp Arg Arg Lys Met Lys Pro Val Gln Phe Val Ala Ala
210 215 220

gtg agt cag aac tgg acc ttt cat ggc cct gga gct agc ggc cag gca 8701
Val Ser Gln Asn Trp Thr Phe His Gly Pro Gly Ala Ser Gly Gln Ala
225 230 235

gct gcc aat tgg cta gca gga ttt gga agg ggt ccc aca cca ccc acc 8749
Ala Ala Asn Trp Leu Ala Gly Phe Gly Arg Gly Pro Thr Pro Pro Thr
240 245 250 255

ttg ctg ggc atc agg caa aat gcg aat acc aca ggc aga agg cgg cta 8797
Leu Leu Gly Ile Arg Gln Asn Ala Asn Thr Thr Gly Arg Arg Arg Leu
260 265 270

ttg gtc ttg gat gag ttc aaa atg gag aaa aga atc agc aga atg ttt 8845
Leu Val Leu Asp Glu Phe Lys Met Glu Lys Arg Ile Ser Arg Met Phe
275 280 285

tat ata atg act ttc ctc ttc cta acc ttg tgg ggt ccc tac ctg gtg 8893

Tyr Ile Met Thr Phe Leu Phe Leu Thr Leu Trp Gly Pro Tyr Leu Val
290 295 300

gcc tgc tat tgg aga gtt ttt gca cga ggg cct gta gta cca ggg gga 8941
Ala Cys Tyr Trp Arg Val Phe Ala Arg Gly Pro Val Val Pro Gly Gly
305 310 315

ttt cta aca gcc gct gtc tgg atg agt ttc gcc caa gca gga atc aat 8989
Phe Leu Thr Ala Ala Val Trp Met Ser Phe Ala Gln Ala Gly Ile Asn
320 325 330 335

ccc ttt gtc tgc att ttc tcc aac agg gag ctg agg cgc tgt ttc agc 9037
Pro Phe Val Cys Ile Phe Ser Asn Arg Glu Leu Arg Arg Cys Phe Ser
340 345 350

aca acc ctt ctt tac tgc aga aaa tcc agg tta cca agg gaa cct tac 9085
Thr Thr Leu Leu Tyr Cys Arg Lys Ser Arg Leu Pro Arg Glu Pro Tyr
355 360 365

tgt gtt ata tgagggagca tctgtaaatc tttagccttg tgaaacacta 9134
Cys Val Ile
370

accctctctg ctaagcaatt gtggcctgta gccatgtctg gagaagaaaa ccaaggatgg 9194

gaatcagcag tgacaaggat ttgggcaaca ttctgcagtc tttgcaatag ttcacctata 9254

atcctatttt aaatctcgga gtgatcctgc tgactgctgg cgaaggtttg taattaagaa 9314

aggacagaac cactgcccta agtttctttc tgtgggtgaa cactagataa cgaaagtagc 9374

aggtgctaag tatcagtgct aaatgctgtg tatatcatta catatgaaaa aaaacttcaa 9434

aaaacaatta gcattggaca ttttaataaa ttaagttgac atgaggtaaa tgtgttgata 9494

aaagctaatt ttagaagttt gaagacttta aaacatttca tactattttt gttttgcaaa 9554

gactacacta ctgggggact taacagtact gtaattctta aaagatgtgc catgaattat 9614

tggggtacac cactttaaac acgccttgta agtttggggg agcattccaa agcagtatat 9674

tggttccaat tagagtttac cttttttgta ttaatatatt gctatttcta aataccactt 9734

tccttcccta ccagtgaaat tgctagcatt gaactgtatt atgtgttttt tgttgattca 9794

gtaaaaaaaa agaaaaagaa aaagtttcca gtttgtttct attttacaaa tgctagatat 9854

ctctctggga ggcaacatga atggaatcaa ctgaccagag atgagcagtt cgaaaaatgc 9914

agaataaaca cgtgttgcct taaagggtta tctagtatcc ttcatcttct ttagcagtgg 9974

agcagtagtc aagaggcaat gaatcagtaa ctggtcctgg ctgctcatcg ggaagtgcat 10034

ggaagagcat tgaatacttg tctaatggtt tggtttggaa attaaggtgc acaccattag 10094

tataatgaga ttttcttctg aggtgtgctg cccattctag tggggtctaa gaagcagaca 10154

gttgatatgt gtttatactg tatgtcagct gtcaggggcg cccacagcct tcgtatgaca 10214

tctggcataa cttgtgaagc atttattgta tggaaggcac agtcttcttt atactttctg 10274

cacattcagt gtattggtcc tttaaattat ttcagtttta acttgtgaaa gcttataata 10334

tgatttctgg tattttagaa atacattaga ttctgtgagt ctcattcttt aagatacaga 10394

tgtgtgaact tcaatataaa gttgcatttg ccaaaattta tctgtgtagc ctgttaattt 10454

ttgtggaata aattttacat atttggcata taattttctt taatttagca tataagcaca 10514

aactagacaa atttgcttta taataatttt atttttataa ctactatatt ttgaaactga 10574

aaatatatga atgatagcaa aaagctgact taatatttaa aagcattatt aagcttttca 10634

aatccattcc tttatttatt attatatgcc attagtattt acaaatgaat atatgtctaa 10694

tggtttggtt tggaatttta atataattat tgcattttaa tgtatagtag aaattttgag 10754

ttctaaatct attgctttta agaaaggctt gtaggctcag tattacaaag ccagagaaag 10814

ttgccattag ttctattgga tttccatagt tttgtttgct tctggaggga ttttgtttgt 10874

ttgtttttct tttgtatagg aaaaataagg ttaagaacct agtaaaacag gatttctcac 10934

tgatatagta tttatataaa cagaatttta atataaatag aaaaaaaaca aataactgac 10994

aatttcaatg tctatgcaac tataaagttt ttgctcaagc cattattagt gagcagtcta 11054

acctcatttc tacaccaaag ctgatttgat ctgggacaaa attgaataca cagaggtgag 11114

gcggatccag caataaaggc tccttgttgg tactagaacc attaccaaaa acacaataaa 11174

aacacggtta ccaaagcatg atctgctaac atatttccat ctcaaaattt gcaggagaga 11234

cacatgacat tgattatttt catgacgaac tttgcttaaa ggttcaaata tcaatcagaa 11294

atgtcagcaa tgatcttgag gccagcaaac ttgggtttca ccaacccata cttccattac 11354

ttagataagt gaacaggtgt ggatcaaggg gattggtgat agcacaaaag agaaagacaa 11414

gtgattttgc ctgactccac ctagctactt ggactataac tcagtttgga ttagtgactg 11474

gtcccaggag gggattagtg aacacagtta actctagtga ctgtctctct tgaacaactt 11534

gaaattttca gatgaaggct gaatcagtgg atcagtcaca gcctcagcta ccaatcaggt 11594

ttttaagcca gtaaataatc gtagtaataa aaaaaagtaa aagtcttccc cagggtcttt 11654

atgacaaaaa attttgctat tttggtaacc ctttccgtag tcaataaaaa cagttggtaa 11714

ttaattcaac ttgaaaaggt tactaatcct caatccataa aaatacattc tcactgggga 11774

gtccaacttc ccatggagag agactcctca tcaataaata atgggctctt cttggactaa 11834

ttacactaca gagctgaaca gtcttcacat ggtttttcca tcagagtggt aatgtttttc 11894

ggtggatatg cctgtgctct gccatctggt tcactttgta cacagtagat ttttatgttc 11954

atggctgtgg aatcccatgc agatattaac taaggcagat ggaggtgagt cgcttgtctg 12014

tttttctaca taggcttgaa ttaaataaca aaatggatta taattgaaaa acattcattg 12074

taaataggaa agtcaattga ctatcttctt tacatactag ctaactgaat ttcaacatca 12134

tcatactcta tagaattaac tgatctgtca ttgttaacaa ttggaaatag ccacatttct 12194

atggatgctc atctgaatag tctcctcatt tacctagtaa atctgttcca agctctgggt 12254

ttcatgttgt gagtactgtg gaaaacatga cagaatgatc tcttattgag cttagacacc 12314

tgggaaagga tcaacagata tggagtaaca gtcgtgtgtg ctaaaatcat aaatactcct 12374

agagcagcat cagaattaat gagcacaaat taagtgtttg caccttctct ctaatgcctt 12434

atctcctggt ttgccttgga atcttttata tgtcctactt tgtcaactct gggaagacgt 12494

ttgctactta ctaaggaaaa tgtcaggatc tagagaaaaa acctcccaca cctccccctg 12554

aacctgaaac ataaaatgaa tgcaattgtt gttgttaact tgtttattgc agcttataat 12614

ggttacaaat aaagcaatag catcacaaat ttcacaaata aagcattttt ttcactgcat 12674

tctagttgtg gtttgtccaa actcatcaat gtatcttatc atgtctggat ccccgcggcc 12734

gcggtacc 12742

<210> 2
<211> 370
<212> PRT
<213> Mus sp.

<400> 2

Met Ala Asn Tyr Ser His Ala Ala Asp Asn Ile Leu Gln Asn Leu Ser
1 5 10 15

Pro Leu Thr Ala Phe Leu Lys Leu Thr Ser Leu Gly Phe Ile Ile Gly
20 25 30

Val Ser Val Val Gly Asn Leu Leu Ile Ser Ile Leu Leu Val Lys Asp
35 40 45

Lys Thr Leu His Arg Ala Pro Tyr Tyr Phe Leu Leu Asp Leu Cys Cys
50 55 60

Ser Asp Ile Leu Arg Ser Ala Ile Cys Phe Pro Phe Val Phe Asn Ser
65 70 75 80

Val Lys Asn Gly Ser Thr Trp Thr Tyr Gly Thr Leu Thr Cys Lys Val
85 90 95

Ile Ala Phe Leu Gly Val Leu Ser Cys Phe His Thr Ala Phe Met Leu
100 105 110

Phe Cys Ile Ser Val Thr Arg Tyr Leu Ala Ile Ala His His Arg Phe
115 120 125

Tyr Thr Lys Arg Leu Thr Phe Trp Thr Cys Leu Ala Val Ile Cys Met
130 135 140

Val Trp Thr Leu Ser Val Ala Met Ala Phe Pro Pro Val Leu Asp Val
145 150 155 160

Gly Thr Tyr Ser Phe Ile Arg Glu Glu Asp Gln Cys Thr Phe Gln His
165 170 175

Arg Ser Phe Arg Ala Asn Asp Ser Leu Gly Phe Met Leu Leu Leu Ala
180 185 190

Leu Ile Leu Leu Ala Thr Gln Leu Val Tyr Leu Lys Leu Ile Phe Phe
195 200 205

Val His Asp Arg Arg Lys Met Lys Pro Val Gln Phe Val Ala Ala Val
210 215 220

Ser Gln Asn Trp Thr Phe His Gly Pro Gly Ala Ser Gly Gln Ala Ala
225 230 235 240

Ala Asn Trp Leu Ala Gly Phe Gly Arg Gly Pro Thr Pro Pro Thr Leu

245 250 255

Leu Gly Ile Arg Gln Asn Ala Asn Thr Thr Gly Arg Arg Arg Leu Leu
260 265 270

Val Leu Asp Glu Phe Lys Met Glu Lys Arg Ile Ser Arg Met Phe Tyr
275 280 285

Ile Met Thr Phe Leu Phe Leu Thr Leu Trp Gly Pro Tyr Leu Val Ala
290 295 300

Cys Tyr Trp Arg Val Phe Ala Arg Gly Pro Val Val Pro Gly Gly Phe
305 310 315 320

Leu Thr Ala Ala Val Trp Met Ser Phe Ala Gln Ala Gly Ile Asn Pro
325 330 335

Phe Val Cys Ile Phe Ser Asn Arg Glu Leu Arg Arg Cys Phe Ser Thr

340 345 350

Thr Leu Leu Tyr Cys Arg Lys Ser Arg Leu Pro Arg Glu Pro Tyr Cys
355 360 365

Val Ile
370

<210> 3
<211> 40
<212> DNA
<213> Artificial Sequence

<220>
<223> Description of Artificial Sequence: an artificially synthesized primer sequence

<400> 3
attcgacgtc gatctttttt ccgtaaactc aataccaggc 40

<210> 4
<211> 20
<212> DNA
<213> Artificial Sequence

<220>
<223> Description of Artificial Sequence: an artificially synthesized primer sequence

<400> 4
gcgggcatca aggagtcaag 20

<210> 5
<211> 20
<212> DNA
<213> Artificial Sequence

<220>
<223> Description of Artificial Sequence: an artificially synthesized primer sequence

<400> 5
ctcctgtccc tcccgttgac 20

<210> 6
<211> 29
<212> DNA
<213> Artificial Sequence

<220>
<223> Description of Artificial Sequence: an artificially synthesized primer sequence

<400> 6
acgcgtcgac ctgcccgtgc tcctgagtg 29

<210> 7
<211> 29
<212> DNA
<213> Artificial Sequence

<220>
<223> Description of Artificial Sequence: an artificially synthesized primer sequence

<400> 7
acgcgtcgac ccaagctctg aaaaaccag 29

<210> 8
<211> 36
<212> DNA
<213> Artificial Sequence

<220>
<223> Description of Artificial Sequence: an artificially synthesized primer sequence

<400> 8
ggggtaccgc ggccgcgggg atccagacat gataag 36

<210> 9
<211> 44
<212> DNA
<213> Artificial Sequence

<220>
<223> Description of Artificial Sequence: an artificially synthesized

primer sequence

<400> 9
ctagtctaga gtcgacatgg cgaactatag ccatgcagcc gaca 44

<210> 10
<211> 36
<212> DNA
<213> Artificial Sequence

<220>
<223> Description of Artificial Sequence: an artificially synthesized primer sequence

<400> 10
ctagtctaga tcctgacatt ttccttagta agtagc 36

<210> 11
<211> 20
<212> DNA
<213> Artificial Sequence

<220>
<223> Description of Artificial Sequence: an artificially synthesized primer sequence

<400> 11
gcacgagggc ctgtagtacc 20

<210> 12
<211> 20
<212> DNA
<213> Artificial Sequence

<220>
<223> Description of Artificial Sequence: an artificially synthesized primer sequence

<400> 12
catccagaca gcggctgtta 20

## Description

TECHNICAL FIELD

The present invention relates to a model animal which may be utilized as a useful tool in screening for a therapeutic agent for schizophrenia, and a novel method of screening for a therapeutic agent for schizophrenia using the model animal.

BACKGROUND ART

Schizophrenia is a chronic, severe, and disabling brain disease showing positive symptoms such as hallucinations and delusions, negative symptoms such as social withdrawal and flat affect, and cognitive impairment. Although 1% of the population develops schizophrenia during their lifetime regardless of race and sex, a monozygotic (identical) twin of a person with schizophrenia has a 40-50% risk of developing the illness, and a child whose parent has schizophrenia has a risk of 6-17%. These data shows genetic factors are associated with the development of schizophrenia. Further, it is considered from the inconsistency between monozygotic twins that the development of schizophrenia may be involved in other factors such as environment and development (nonpatent reference 1).
Recent studies propose plural hypotheses about the development of schizophrenia. The relationship between dopamine and schizophrenia (dopamine hypothesis) was found in clarifying the action mechanisms of therapeutic agents for schizophrenia. Further, a possibility in which a neurotransmission by glutamic acid, a neurotransmitter, might be involved in the development of schizophrenia (glutamic acid hypothesis) was suggested, from a result in which an administration of a blocker against the neurotransmitter glutamic acid, such as PCP (phencyclidine), promoted symptoms such as hallucinations and delusions. Furthermore, a hypothesis in which a disorder in forming the neural network during the development stage may be involved in the development of schizophrenia has been proposed (developmental disorder hypothesis).
Therapeutic agents for schizophrenia have been used from the 1950s. Conventional medicaments for schizophrenia, which are based on the blocking effect of dopamine on the neurotransmission, generally have a therapeutic effect on positive symptoms such as hallucinations and delusions, but a little or no effect on negative symptoms and cognitive impairment, and it is known that these medicaments cause adverse effects by blocking the dopamine neurotransmission, particularly movement disorders (Lieberman et al., N Engl J Med., 353, 1209-1223, 2005). All medicaments now used have the activity of blocking the dopamine neurotransmission, but the above problems remain. A more effective drug having fewer side effects, based on a new action mechanism, has been desired (non-patent reference 2).
To find a potent drug, not only the effect of the drug on the target molecule thereof, but also the bioavailability and the transitional property into the brain of the drug in vivo, should be taken in consideration. A model animal which makes an analysis at the individual level possible is necessary, for example, to clarify the mechanism of development, to prevent the development, to alleviate the symptoms, and to develop agents and medical technologies for treatment.
As a model animal of schizophrenia, there may be mentioned, for example, an animal to which a dopaminergic agent is administered or an animal to which a blocker against the transmission of glutamic acid is administered, on the basis of the dopamine hypothesis or glutamic acid hypothesis, or an animal in which the hippocampus is destroyed, on the basis of the developmental disorder hypothesis. However, there is a strong perception that these model animals do not reflect genetic backgrounds which would cause the development of schizophrenia, but merely reflect a part of phenotypes disordered in schizophrenia, particularly positive symptoms (non-patent reference 3).
Transgenic animals are desired as a method of studying functions of genes in a living body, or as a model animal for developing therapeutic agents. However, it is difficult to prepare a model reflecting a human disease having a mechanism of development which is unknown, such as schizophrenia.
SREB2 is disclosed as a novel protein which belongs to a G protein-coupled receptor family and is mainly expressed in the central nervous system and the urinary and genital organs (patent reference 1, non-patent reference 4). It was reported that percentages of specific haplotypes defined by SNPs of SREB2 in patients with schizophrenia were higher than those in controls without schizophrenia (patent reference 2). However, an SREB2 transgenic animal has not been prepared, and it was not known that SREB2 is a factor of schizophrenia.
[patent reference 1] WO 99/46378
[patent reference 2] WO 02/086147
[non-patent reference 1] Melissa K. Speaking, Schizophrenia, [online], August, 2002, National Institute of Mental Health, [retrieved on July 14, 2005], internet <URL: http://www.nimh.nih.gov/publicat/NIMHschizoph.pdf>
[non-patent reference 2] The New England Journal of Medicine, the U.S.A., 2005, Vol.353, p.1209-1223
[non-patent reference 3] Behavioural Pharmacology, the United Kingdom, 2000, Vol.11, p.223-233
[non-patent reference 4] Biochemical and Biophysical Research Communications, the U.S.A., 2000, Vol.272, p.576-582

## DISCLOSURE OF INVENTION

## PROBLEMS TO BE SOLVED BY THE INVENTION

An object of the present invention is to provide a model animal which exhibits schizophrenia, particularly negative symptoms and cognitive impairment, and reflects the genetic background that causes schizophrenia, and an in vivo screening method for a therapeutic agent for schizophrenia.

## MEANS FOR SOLVING THE PROBLEMS

As a result of intensive studies, the present inventors prepared transgenic mice in which SREB2 belonging to a G protein-coupled receptor family was overexpressed using an α-calcium-calmodulin-dependent kinase II promoter region capable of selectively expressing a gene of interest in the brain, particularly the forebrain (including the cerebral cortex and hippocampus). The transgenic mice unexpectedly exhibited schizophrenic symptoms, and it was found that the overexpression of SREB2 in the brain caused schizophrenia. Further, the present inventors found that the transgenic mice may be used as a model animal of schizophrenia that is a screening tool for a therapeutic agent for schizophrenia, and constructed an in vivo screening system for a therapeutic agent for schizophrenia using an alleviation of schizophrenia-related disorders in the transgenic mice as an index. Furthermore, the present inventors found that the transgenic mice exhibited the negative symptoms and cognitive impairment of schizophrenia. The present inventors provided SREB2 transgenic animals as a model animal that was a tool useful in screening for a therapeutic agent for schizophrenia, particularly negative symptoms and cognitive impairment, and a screening method for a therapeutic agent for schizophrenia using the transgenic animals, and completed the present invention.

The present invention relates to

[1] a non-human transgenic animal exhibiting schizophrenic symptoms, wherein (1) a polynucleotide encoding a polypeptide comprising the amino acid sequence of SEQ ID NO: 2, and (2) a polynucleotide comprising a promoter are introduced,
[2] the non-human transgenic animal of [1], wherein the promoter is an exogenous promoter,
[3] the non-human transgenic animal of [1] or [2], wherein the polypeptide consists of the amino acid sequence of SEQ ID NO: 2,
[4] the non-human transgenic animal of any one of [1] to [3], wherein the promoter is a promoter capable of specifically expressing the polypeptide in the brain,
[5] the non-human transgenic animal of any one of [1] to [4], wherein the promoter is an α-calcium-calmodulin-dependent kinase II promoter,
[6] the non-human transgenic animal of any one of [1] to [5], wherein the animal is a mouse,
[7] the non-human transgenic animal of any one of [1] to [6], wherein an amount of an mRNA expressed in the forebrain is 1.5 times or more, and less than 3 times in comparison with that in a wild type, the mRNA encoding a polypeptide comprising the amino acid sequence of SEQ ID NO: 2,
[8] a model animal of schizophrenia, which is the non-human transgenic animal of any one of [1] to [7],
[9] the model animal of schizophrenia of [8], which is a model animal of a negative symptom and/or cognitive impairment,
[10] use of the non-human transgenic animal of any one of [1] to [7] in the detection of a therapeutic effect on schizophrenia,
[11] the use of [10], wherein the therapeutic effect on schizophrenia is a therapeutic effect on a negative symptom and/or cognitive impairment in schizophrenia,
[12] a method of detecting a therapeutic effect of a test substance on schizophrenia, comprising the steps of:

administrating a test substance to the non-human transgenic animal of any one of [1] to [7], and
analyzing a schizophrenia-related disorder in the animal,

[13] the method of [12], wherein the analyzing step is a step of analyzing an alleviatory effect on a negative symptom and/or cognitive impairment in schizophrenia, and the therapeutic effect on schizophrenia is an alleviatory effect on a negative symptom and/or cognitive impairment,
[14] use of the non-human transgenic animal of any one of [1] to [7] in the screening for a therapeutic agent for schizophrenia,
[15] the use of [14], wherein the therapeutic agent for schizophrenia is a therapeutic agent for a negative symptom and/or cognitive impairment,
[16] a method of screening for a therapeutic agent for schizophrenia, comprising the steps of:

administrating a test substance to the non-human transgenic animal of any one of [1] to [7],
analyzing a schizophrenia-related disorder in the animal, and
selecting the substance exhibiting a therapeutic effect on schizophrenia,

[17] the method of [16], wherein the analyzing step is a step of analyzing an alleviatory effect on a negative symptom and/or cognitive impairment in schizophrenia, the selecting step is a step of selecting the substance exhibiting an alleviatory effect on a negative symptom and/or cognitive impairment in schizophrenia, and the therapeutic agent for schizophrenia is a therapeutic agent for a negative symptom and/or cognitive impairment, and
[18] a method of producing a pharmaceutical composition for treating schizophrenia, comprising the steps of:

screening a test substance by the method of [16] or [17], and
preparing a medicament containing the substance obtained by the screening.

The term "exogenous promoter" as used herein means a promoter other than the SREB2 gene promoter. The term "to exhibit schizophrenic symptoms" means, but is by no means limited to, particularly to show a reduction in prepulse inhibition in the startle response analysis as described below. The term "model animal of schizophrenia" means an animal which may be used in detecting an effect of a test substance on the treatment for schizophrenia or screening for an agent for treating schizophrenia. The term "negative symptoms" means, but is by no means limited to, particularly to exhibit disorders of social behavior in the social behavior test as described below, or to exhibit an extension of the immobility time during swimming in the Morris water maze test as described below. The term "cognitive impairment" means, but is by no means limited to, particularly to exhibit memory and learning disorders in the Morris water maze test and/or the fear conditioning test as described below.

## EFFECTS OF THE INVENTION

Known model animals which had been reported to exhibit schizophrenia-like behavior do not reflect the genetic background that is a factor in developing schizophrenia in patients. The SREB2 non-human transgenic animal of the present invention first enables screening for a true drug having a therapeutic effect on schizophrenia in a schizophrenic model which reflects genetic mutations as a factor of the development in humans. It was confirmed that the percentages of specific haplotypes defined by SNPs of SREB2 were higher in patients with schizophrenia, but functional viewpoints of SREB2, such as a promotion or reduction in the expression, were not concretely disclosed (WO02/086147), and it was unclear what symptoms are caused by the overexpression of SREB2. According to the SREB2 non-human transgenic animal of the present invention, it was first clarified that the overexpression causes schizophrenia. The SREB2 non-human transgenic animal of the present invention exhibits negative symptoms and cognitive impairment of schizophrenia which had not been expressed in known drug-induced models of schizophrenia, and thus, can be utilized as a useful model capable of evaluating negative symptoms and cognitive impairment, the evaluation of which had been difficult. An SREB2 non-human transgenic animal having the introduced gene heterogeneously, as a preferred embodiment of the SREB2 non-human transgenic animal of the present invention, exhibits the schizophrenia-related phenotypes due to the heterogeneity of the introduced gene, and thus, can be easily bred in comparison with other genetically modified animals, particularly knock-out animals, and is a model animal useful in the screening for a drug having a therapeutic effect on schizophrenia.

## BRIEF DESCRIPTION OF THE DRAWINGS

Figure 1 is an illustration showing the procedures of a prepulse inhibition test.
Figure 2 is a graph showing the result of the prepulse inhibition test.
Figure 3 is a graph showing the result (goal latency to the platform during training trials) of a Morris water maze test.
Figure 4 is a graph showing the result (immobility time during training trials) of the Morris water maze test.

## BEST MODE FOR CARRYING OUT THE INVENTION

Terms as used herein will be explained.
The term "receptor" means a receptor protein, and the term "SREB2" means an SREB2 protein.
The term "polynucleotide to be introduced" means a polynucleotide which may be used in preparing a transgenic animal and comprises a promoter region and a polynucleotide encoding a receptor.
Hereinafter the present invention will be further illustrated.

[1] Receptor encoded by a polynucleotide contained in polynucleotide to be introduced, and the polynucleotide encoding the receptor

1) Schizophrenia-related receptor

As a receptor encoded by a polynucleotide contained in a polynucleotide to be introduced for preparing the transgenic animal of the present invention, a polypeptide comprising the amino acid sequence of SEQ ID NO: 2 is used, and a polypeptide consisting of the amino acid sequence of SEQ ID NO: 2 is preferred.

Hereinafter the polypeptide comprising the amino acid sequence of SEQ ID NO: 2 is referred to as "schizophrenia-related receptor".

The amino acid sequence of SEQ ID NO: 2 is that of mouse SREB2 (WO99/46378) belonging to a G protein-coupled receptor family. The amino acid sequences of mouse SREB2, human SREB2, and rat SREB2 are completely identical with each other.

2) Polynucleotide contained in polynucleotide to be introduced

A polynucleotide encoding the receptor, contained in the polynucleotide to be introduced, is not particularly limited, so long as it encodes the schizophrenia-related receptor. The polynucleotide encoding the receptor is preferably a polynucleotide encoding the amino acid sequence of SEQ ID NO: 2, more preferably a polynucleotide consisting of nucleotides 7985-9094 of SEQ ID NO: 1.

[2] Method of preparing transgenic animal

1) Preparation of promoter contained in polynucleotide to be introduced

The polynucleotide to be introduced contains a promoter sequence capable of controlling the expression of the schizophrenia-related receptor and, if desired, may further contain an enhancer sequence. The schizophrenia-related receptor may be expressed systemically or in a specific tissue by selecting appropriate promoter and enhancer sequences. The promoter for preparing the model animal of schizophrenia of the present invention is not particularly limited, but is preferably an exogenous promoter, such as a promoter region of an α-calcium-calmodulin-dependent kinase II (α-CaM-kinase II) gene [Mayford, M. et al., (1990) Proc. Natl. Acad. Sci. USA 93:13250-13255], a promoter region of a neuron-specific enolase [Quon, D. et al., (1991) Nature 352, 239-241], or a promoter region of a Thy-1 gene [Vidal, M. et al., (1990) EMBO J 9:833-840], more preferably a promoter capable of brain-specifically expressing the schizophrenia-related receptor, most preferably a promoter region of an α-calcium-calmodulin-dependent kinase II gene. The α-calcium-calmodulin-dependent kinase II gene is a neuronal gene specifically expressed in the brain, particularly the forebrain. Therefore, a desired gene can be selectively expressed in the brain, particularly the forebrain (including the cerebral cortex and hippocampus), by using the promoter region of an α-calcium-calmodulin-dependent kinase II gene, and as a result, transgenic mice exhibiting schizophrenic symptoms unexpectedly obtained, and it was found that the overexpression of SREB2 in the brain caused schizophrenia.

To prepare the promoter region of an α-calcium-calmodulin-dependent kinase II gene, a primer set can be designed on the basis of the nucleotide sequence (Accession No. AJ222796) registered in a genetic database, GenBank. A polymerase chain reaction (PCR) [Saiki, R. K. et al., (1988) Science 239, 487-491] may be carried out using the designed primer set and a genomic DNA as a template to obtain a part of the polynucleotide to be introduced. As the genomic DNA, a commercially available genomic DNA (Clontech) may be used. Alternatively, a genomic DNA may be obtained from animal blood by using a commercially available genomic DNA extraction kit (Qiagen). The DNA obtained by the PCR may be incorporated into an appropriate vector to obtain the promoter region of α-calcium-calmodulin-dependent kinase II.

2) Preparation of polynucleotide encoding schizophrenia-related receptor

A polynucleotide encoding the schizophrenia-related receptor, contained in the polynucleotide to be introduced, may be obtained in accordance with methods described in WO99/46378. For example, the SREB2 gene consisting of nucleotides 7985-9094 of SEQ ID NO: 1 may be prepared by, for example, a method in which an oligonucleotide probe is synthesized based on a partial sequence of a known nucleotide sequence (WO99/46378) and a cDNA library is screened using this probe, or a method in which an oligonucleotide primer set which hybridize at both ends of a cDNA fragment of interest is synthesized and a reverse transcriptase-polymerase chain reaction (RT-PCR) is carried out using this primer set and mRNAs isolated from cells.

3) Preparation of polynucleotide to be introduced

The polynucleotide to be introduced for preparing the transgenic animal of the present invention contains at least a desired promoter region (preferably an exogenous promoter region) and a polynucleotide encoding the schizophrenia-related receptor. The order of the promoter and the polynucleotide encoding the receptor arranged in the polynucleotide to be introduced is not particularly limited, so long as the polynucleotide encoding the receptor is arranged under the control of the promoter activity. Such a polynucleotide to be introduced may be prepared by sequentially incorporating a promoter region followed by a schizophrenia-related receptor gene into the multicloning site of an appropriate vector, for example, as described in Example 1. As the vector, for example, pUC 18 (Toyobo) may be used. More particularly, a polynucleotide to be introduced may be obtained by the method described in Example 1. As such a polynucleotide to be introduced, there may be mentioned, for example, a gene in which an SREB2 DNA (nucleotides 7985-9094 of SEQ ID NO: 1) and an SV40 poly (A) additional signal are linked to the downstream region of the promoter of an $\alpha$-calcium-calmodulin-dependent kinase II gene, most preferably a polynucleotide consisting of the nucleotide sequence of SEQ ID NO: 1. The SV40 poly (A) additional signal may be commercially available from, for example, pcDNA3.1 (Invitrogen). The method of producing the polynucleotide to be introduced is not particularly limited, but a method using PCR may be utilized. Gene manipulation techniques used in the present invention may be carried out in accordance with conventional methods, such as Maniatis, T. et al., "Molecular Cloning-A Laboratory Manual", Cold Spring Harbor Laboratory, NY, 1982.

4) Preparation of transgenic animal

The transgenic animal of the present invention is not particularly limited, so long as it is a non-human transgenic animal into which a polynucleotide comprising a polynucleotide encoding a schizophrenia-related receptor and a promoter (preferably an exogenous promoter) is introduced, and it exhibits schizophrenia. The transgenic animal of the present invention may be prepared in accordance with a known conventional method (for example, Animal Biotechnology, 1, 175-84, 1990), except that the above-mentioned polynucleotide to be introduced is used. It may be prepared in accordance with, for example, the procedures described in Example 1 below. That is, a transgenic animal of interest may be prepared by introducing the polypeptide to be introduced into each totipotent cell of a non-human animal, allowing these cells to develop into individuals, and selecting an individual in which the polynucleotide is integrated into the genome of somatic cells. The term "transgenic animal" as used herein means a transgenic animal excluding a human (i.e., a non-human transgenic animal), such as mammals other than a human (for example, rat, mouse, dog, cat, monkey, pig, cattle, sheep, rabbit, goat, dolphin, or horse), birds (for example, chicken or quail), amphibians (for example, frog), reptiles, or insects (for example, Drosophila). As the non-human animal, it is technically possible to use all animal species, but rodents are preferable, a mouse or a rat is more preferable, a mouse is most preferable. In the case of a mouse, a large number of inbred strains are created, and fertilized egg culturing, in vitro fertilization and the like techniques are well established. As the totipotent cell into which a gene is introduced, a fertilized egg or an early stage embryo can be used in the case of a mouse. A physical injection (microinjection) of DNA is preferred as a method for introducing the gene into a cultured cell, when the production efficiency of transgenic animal individuals and the transmission efficiency of the introduced gene to the next generation are taken into consideration.

For example, a vector dissolved in an HEPES buffer, a phosphate buffer, a physiological saline or the like is injected into a fertilized egg with a micropipette, and this egg is transplanted into the uterus of a pseudopregnant host animal previously treated with a hormone [such as prostaglandin (PG) $F_2\alpha$, human chorionic gonadotropin (hCG), estradiol, luteinizing hormone (LH) or the like] or by a physical stimulation (in the case of a small animal). By feeding this host animal and allowing it to give birth, a gene-introduced non-human animal may be obtained. Whether or not a gene-introduced non-human animal is obtained may be determined by extracting DNA from a part of the body (for example, the tail tip) and confirming the presence of the introduced polynucleotide by the Southern analysis or PCR. When an individual in which the presence of the introduced polynucleotide was confirmed is regarded as the founder, the introduced polynucleotide is transmitted to 50% of its offspring, and it is possible to efficiently prepare wild type and mutation type animals. As a model animal of schizophrenia, particularly a model animal of negative symptoms and/or cognitive impairment in schizophrenia, a model animal (particularly a model mouse) in which an amount of mRNA encoding a schizophrenia-related receptor (particularly SREB2) and expressed in the forebrain (including the cerebral cortex and hippocampus) is 1.5 times or more, and less than 3 times in comparison with that in a wild type, is more preferable, and a model animal (particularly a model mouse) in which an amount of mRNA of a schizophrenia-related receptor (particularly SREB2) is 1.5 times or more, and less than 2.5 times at the age of 7-weeks, in comparison with that in a wild type, is most preferable. The amount of SREB2 mRNA expressed can be determined by the method described in Example 3.

The transgenic animal of the present invention, including those prepared as described above and its offspring exhibiting schizophrenic symptoms, is useful in detecting a therapeutic effect on schizophrenia (particularly a therapeutic effect on negative symptoms and/or cognitive impairment), and screening for a therapeutic agent for schizophrenia (particularly a therapeutic agent for negative symptoms and/or cognitive impairment).

[3] Method of measuring schizophrenia-related disorders and method of detecting therapeutic effect on schizophrenia

It is possible to detect whether or not a test substance has an effect for treating (curing or alleviating) schizophrenia-related disorders in the transgenic animal and to detect a therapeutic effect of the test substance on schizophrenia, by conventional methods of measuring schizophrenia-related disorders, such as the following methods described in items 1) to 4), based on the alleviation of the schizophrenia-related disorders (for example, an inhibitory effect on a reduction in prepulse inhibition in a startle response analysis, an alleviative effect on a social behavior disorder in a social behavior test, or an alleviative effect on memory and learning disorders in a Morris water maze test and/or a fear conditioning test) as an index.
Further, it is possible to detect whether or not a test substance has an effect for treating (curing or alleviating) negative symptoms of schizophrenia in the transgenic animal and to detect a therapeutic effect of the test substance on negative symptoms of schizophrenia, by the methods described in items 2) and 3), based on the alleviation of negative symptoms of schizophrenia (for example, an alleviative effect on a social behavior disorder in a social behavior test, or an alleviation of immobility in a Morris water maze test) as an index.
Furthermore, it is possible to detect whether or not a test substance has an effect for treating (curing or alleviating) cognitive impairment of schizophrenia in the transgenic animal and to detect a therapeutic effect of the test substance on cognitive impairment of schizophrenia, by the methods described in items 3) and 4), based on the alleviation of cognitive impairment of schizophrenia (for example, an alleviative effect on memory and learning disorders in a Morris water maze test and/or a fear conditioning test) as an index.

1) Startle response analysis (prepulse inhibition test)

As a measuring system, a startle response system for small animals (SR-LAB; San Diego Instruments) composed of a sound-proof cabinet in which a retaining cylinder affixed with a vibration sensor is arranged, a speaker for loading sound stimuli, and a control computer is used.
Startle responses of a test animal against pulses are measured as amplitude via the vibration sensor. A background white noise of 70 dB is always loaded during the measurement to insulate the animal from the outside world. Each of wild type (WT) mice and transgenic (Tg) mice is put into the startle response system. After an acclimation for 3 minutes, each of the following 6 stimuli a) to f) is randomly loaded six times (36 stimuli in total) at intervals of approximately 15 seconds, and each vibration of the cylindrical enclosure after 65 msec from each sound stimulus is measured to carry out the following evaluation. The interval between the beginning of prepulse loading and the beginning of pulse loading is 100 msec. The abbreviations P and PP mean pulse and prepulse, respectively, and the vertical axis indicates amplitude.
a) Non-stimulus (background white noise): BG
b) 80 dB (40 ms) (prepulse alone): PP
c) 120 dB (40 ms) (pulse alone): P
d) Prepulse of 73 dB (20 ms) + pulse of 120 dB (40 ms): 3D
e) Prepulse of 76 dB (20 ms) + pulse of 120 dB (40 ms): 6D
f) Prepulse of 82 dB (20 ms) + pulse of 120 dB (40 ms): 12D

The items to be measured are as follows:

i) A startle response when a single pulse of 120 dB is loaded [the above item c)] [vibration coefficient: a mean value (N=6) of the value A as shown in Figure 1].
ii) In the above three prepulse conditions d) to f), each PPI (prepulse inhibition) defined as the following equation is calculated from each mean value (N=6):

$$PPI\ (\%) = [1-(B/A)] \times 100$$

wherein the abbreviations A and B are values A and B shown in Figure 1, respectively.
iii) Vibration coefficients [mean values (N=6)] when no stimulus is loaded [the above item a)] and a single prepulse is loaded [the above item b)].

When prepulse inhibition is lowered, it is regarded as exhibiting schizophrenic symptoms.

2) Social behavior test

As social behavior of two individuals, two mice are brought into contact with each other in a new plastic cage (PC) for 60 minutes, and the frequencies and accumulated times of social investigation, following, and resting are measured. Further, the behavior of the mice is recorded with a video camera, and loaded on DVD-Rs using a DVD recorder. This observation of behavior is carried out, for example, from 9 p.m. to 12 p.m. under room light. When reductions in the frequencies and/or the accumulated times of social investigation and/or following are observed, it is regarded as exhibiting negative symptoms of schizophrenia.

As social behavior of a group, behavior in a cage is recorded with a video camera, and loaded on DVD-Rs using a DVD recorder. This recording begins, for example, from 9 p.m. to 12 p.m. and is continued for 48 hours.

3) Morris water maze test

a) Setting of apparatus

As an apparatus for this test, a platform (diameter = approximately 10 cm, height = approximately 26.5 cm) of transparent acrylic resin that is not optically recognized, and a circular pool (diameter = approximately 68 cm, height = approximately 32 cm) of gray vinyl chloride resin are used. This pool is filled with water (water temperature = 17 to 18°C) up to a height of approximately 27.5 cm so that the platform is hidden in water. A black square pole is put up on the platform to indicate the position thereof. The pool is divided into quadrants, and the platform is set at the center of the fourth quadrant (approximately 17 cm from the center of the pool). A red triangular pyramid is placed around the pool as a spatial clue.

The swimming behavior of each mouse is analyzed using a video-tracking system (SMART; Panlab), and loaded on DVD-Rs using a DVD recorder. The data loaded on DVD-Rs are used for reference, and the results printed out from the video-tracking system are used as primary data.

b) Measuring methods

(1) Training trial

Training trials are carried out twice a day (morning and afternoon) for 4 days (8 trials in total per mouse). The black square pole is put up on the platform on the first and second days of the training, and the training is carried out without the pole on the third and fourth days.

A mouse is put into the pool so that the head thereof is directed to the wall of the circular pool, and a goal latency (seconds) to the platform is measured with a stopwatch. The upper limit of the goal latency is 90 seconds. When a mouse reaches the platform in 90 seconds or less and stays on the platform for 30 seconds, it is judged that the mouse recognizes the position of the platform, and the measurement is stopped. When a mouse does not reach the platform, the goal latency of the mouse is regarded as 90 seconds.

In this connection, when a mouse does not reach the platform on the first day, the mouse is placed on the platform for 30 seconds after the measurement, and returned to the cage. When a mouse does not reach the platform in 90 seconds on the second to fourth days, the mouse is directly returned to the cage. When a mouse reaches the platform but returns to the pool during the measurement, it is judged that the mouse does not recognize the position of the platform, and the measurement is continued. The trials on the second to fourth days should be carried out at the same time as that of the trial on the first day whenever possible.

(2) Probe trial (observation of staying time when the platform is removed: confirmation of spatial cognition and acquisition of position learning in animal)

A probe trial is carried out in the morning of the fifth day. A mouse put into the pool so that the head thereof is directed to the wall of the circular pool. In measuring periods of 0 to 30 seconds, 30 to 60 seconds, and 60 to 90 seconds, the staying times in the fourth quadrant (i.e., the quadrant in which the platform is set during the training trials) (swimming times in the fourth quadrant: seconds) and the frequencies of passing through the position in which the platform had been set in the fourth quadrant (frequencies of passing through the position in which the platform had been set) are measured.

When an extension of the goal latency to the platform during the training trials, and/or a shortening of the swimming time in the fourth quadrant and/or a reduction in the frequency of passing through the fourth quadrant during the probe trail are observed, it is judged that the mouse has memory and learning disorders, and is regarded as expressing cognitive impairment of schizophrenia.
When no behavior is observed during the swimming, it is judged that the mouse shows immobilization, and is regarded as expressing negative symptoms of schizophrenia.

4) Fear conditioning test

A mouse is put into a fear conditioning test box (Acti Metrics), and electric stimuli are loaded for fear conditioning. Electric stimuli of 0.4 mA for 1 second are loaded at intervals of 15 seconds for 4 minutes.
Next day, the mouse is put into the fear condition analysis system again, and is observed for 4 minutes under conditions without electric shocks to measure an immobility time.
When the immobility time is shortened, it is judged that the mouse has memory and learning disorders, and is regarded as expressing cognitive impairment of schizophrenia.

[4] Screening method for therapeutic agent for schizophrenia

A therapeutic agent for schizophrenia can be screened by administering a test substance to the transgenic animal of the present invention, analyzing (preferably measuring) schizophrenia-related disorders in the animal, and selecting the substance having a therapeutic effect on schizophrenia. Further, a therapeutic agent for negative symptoms and/or cognitive impairment of schizophrenia can be screened by administering a test substance to the transgenic animal of the present invention, analyzing (preferably measuring) negative symptoms and/or cognitive impairment in the animal, and selecting the substance having a therapeutic effect on negative symptoms and/or cognitive impairment.
Test substances which may be applied to the screening method of the present invention are not particularly limited, but there may be mentioned, for example, commercially available compounds (including peptides), various known compounds (including peptides) registered in chemical files, compounds obtained by combinatorial chemistry techniques [N.K.Terrett, M.Gardner, D.W.Gordon, R.J.Kobylecki, J.Steele, Tetrahedron, 51, 8135-73(1995)], culture supernatants of microorganisms, natural components derived from plants or marine organisms, animal tissue extracts, or chemically or biologically modified compounds (including peptides) derived from compounds (including peptides) selected by the screening method of the present invention.

[5] Method of producing pharmaceutical composition for treating schizophrenia

A therapeutic agent for schizophrenia, particularly a therapeutic agent for negative symptoms and/or cognitive impairment, selected by the screening method of the present invention may be used as a main composition to obtain a medicament. This medicament is useful in treating schizophrenia, particularly negative symptoms and/or cognitive impairment.
The medicament comprising a therapeutic agent for schizophrenia as an active ingredient may be prepared using carriers, fillers, and/or other additives generally used in the preparation of medicaments, in accordance with the active ingredient. Examples of administration include oral administration by tablets, pills, capsules, granules, fine granules, powders, oral solutions and the like, and parenteral administration by injections (e.g., intravenous, intramuscular, or the like), suppositories, transdermal preparations, transmucosal absorption preparations and the like. Particularly, in the case of polypeptides which are digested by enzymes, a parenteral administration such as an intravenous injection or the like is desirable.
In the solid composition for use in the oral administration, one or more active substances may be mixed with at least one inert diluent such as lactose, mannitol, glucose, microcrystalline cellulose, hydroxypropylcellulose, starch, polyvinyl pyrrolidone, or aluminum magnesium silicate. In the usual way, the composition may contain additives other than the inert diluent, such as a lubricant, a disintegrating agent, a stabilizing agent, or a solubilizing or solubilization assisting agent. If necessary, tablets or pills may be coated with a sugar coating or a film of a gastric or enteric substance.
The liquid composition for oral administration may include, for example, emulsions, solutions, suspensions, syrups, and elixirs, and may contain a generally used inert diluent such as purified water or ethyl alcohol. The composition may contain additives other than the inert diluent, such as moistening agents, suspending agents, sweeteners, flavors, or antiseptics.
The injections for parenteral administration may include aseptic aqueous or non-aqueous solutions, suspensions, and emulsions. Examples of the diluent for use in the aqueous solutions and suspensions include distilled water for injection use and physiological saline. Examples of the diluent for use in the non-aqueous solutions and suspensions include propylene glycol, polyethylene glycol, plant oil (e.g., olive oil), alcohols (e.g., ethanol), polysorbate 80 and the like. Such a composition may further contain a moistening agent, an emulsifying agent, a dispersing agent, a stabilizing agent, a solubilizing or solubilization assisting agent, an antiseptic or the like. These compositions may be sterilized, for example, by filtration through a bacteria retaining filter, blending of a germicide, or irradiation. Alternatively, these compositions may be used by first making them into sterile solid compositions and dissolving them in sterile water or other sterile solvent for injection prior to their use.

The dose of the medicament comprising as an active ingredient a therapeutic agent for schizophrenia selected by the screening method of the present invention is optionally decided by taking into consideration the strength of each active ingredient used, symptoms, age, sex, or the like of each patient to be administered.

EXAMPLES

The present invention now will be further illustrated by, but is by no means limited to, the following Examples. The following gene manipulations may be performed in accordance with known methods [for example, Maniatis, T. et al. (1982): Molecular Cloning- A Laboratory Manual, Cold Spring Harbor Laboratory, NY)], unless otherwise specified. Commercially available reagents and kits may be used in accordance with protocols attached thereto.

Example 1: Construction of polynucleotide to be introduced for preparing SREB2-overexpressed transgenic mice

In this Example, a polynucleotide to be introduced (SEQ ID NO: 1) for preparing SREB2-overexpressed transgenic mice, consisting of a gene in which an SREB2 DNA and an SV40 poly (A) additional signal are linked to the downstream region of the promoter of an α-calcium-calmodulin-dependent kinase II gene, was prepared.

The promoter region of an α-calcium-calmodulin-dependent kinase II was obtained as two overlapping fragments by performing the following PCR using a genomic DNA from a C57BL/6 mouse as a template. The genomic DNA of a C57BL/6 mouse was purified from mouse blood using a genomic DNA extraction kit (QIAamp DNA Blood Midi Kit; QIAGEN). Primers were designed based on the sequence (Accession No. AJ222796) registered in a gene database GenBank. A forward primer consisting of the nucleotide sequence of SEQ ID NO: 3 and a reverse primer consisting of the nucleotide sequence of SEQ ID NO: 4 were used to obtain a DNA fragment of 4.6 kb. An AatII recognition site was added at the 5'-terminus of this forward primer. Further, a forward primer consisting of the nucleotide sequence of SEQ ID NO: 5 and a reverse primer consisting of the nucleotide sequence of SEQ ID NO: 6 were used to obtain a DNA fragment of 3.7 kb. An SalI recognition site was added at the 5'-terminus of this reverse primer. In each PCR using a DNA polymerase (Pfu Turbo; Stratagene), a reaction at 99°C for 1 minute was carried out and a cycle composed of reactions at 99°C for 15 seconds, at 58°C for 15 seconds, and at 75°C for 10 minutes was repeated 45 times, or a reaction at 95°C for 1 minute was carried out and a cycle composed of reactions at 95°C for 15 seconds, at 62°C for 15 seconds, and at 75°C for 8 minutes was repeated 40 times. The resulting DNA fragment of 4.6 kb was ligated to that of 3.7 kb, using the internal XmaI recognition site located in the overlapping region, to obtain the promoter region of an α-calcium-calmodulin-dependent kinase II. The SV40 poly (A) additional signal was obtained by carrying out a PCR using a plasmid pME18S (Maruyama et al., Shin Seikagaku Jikken Koza, 123-133, 1991) as a template, forward (SEQ ID NO: 7) and reverse (SEQ ID NO: 8) primers, and a DNA polymerase (Pfu Turbo; Stratagene). This reverse primer contained a KpnI recognition site and an NotI recognition site.

The SREB2 DNA was obtained by carrying out a PCR using mouse chromosomes as a template. A forward primer consisting of the nucleotide sequence of SEQ ID NO: 9 was designed on the basis of the upstream sequence of an SREB2 ORF, and a reverse primer of SEQ ID NO: 10 was designed on the basis of the downstream sequence thereof. In this PCR using a DNA polymerase (Pfu Turbo; Stratagene), a reaction at 96°C for 1 minute was carried out and a cycle composed of reactions at 96°C for 15 seconds, at 60°C for 15 seconds, and at 75°C for 8 minutes was repeated 30 times. An SalI recognition site was added at the 5'-terminus of this forward primer, and an XbaI recognition site was added at the 5'-terminus of this reverse primer. The resulting fragment of 4.5 kb was ligated via the SalI recognition site to the previously obtained promoter region of an α-calcium-calmodulin-dependent kinase II, and via the XbaI recognition site to the SV40 poly (A) additional signal, and was cloned into a plasmid pUC18 (Toyobo) which had been digested with AatII and KpnI, to obtain a plasmid pCM-SREB2 carrying a polynucleotide to be introduced (13 kb) for preparing SREB2-overexpressed transgenic mice. The plasmid pCM-SREB2 was digested with restriction enzymes AatII and NotI, to isolate and purify the polynucleotide to be introduced (13 kb).

Example 2: Preparation and identification of SREB2-overexpressed transgenic mice

The polynucleotide to be introduced prepared in Example 1 was microinjected into fertilized eggs taken from F1 hybrid mice of C57BL/6 and DBA2 mice, and the resulting fertilized eggs were transplanted into oviducts of ICR foster mother mice [Hogan, B. et al. (1986). Manipulating the mouse embryo: a laboratory manual, Plainview, New York: Cold Harbor Press]. The pregnant mice were allowed to undergo spontaneous delivery, and the obtained 53 offspring mice were subjected to the identification of transgenic mice.

This identification was carried out to compare the numbers of copies of the SREB2 gene contained in the chromosomes of the offspring. A genomic DNA extraction kit (MagExtractor -Genome-; Toyobo) was used to extract and purify genomic DNAs from the tails of mice. Forward and reverse primers consisting of the nucleotide sequences of SEQ ID NOS: 11 and 12, respectively, were designed on the basis of the SREB2 DNA sequence, and a real-time quantitative PCR [PRISM (Registered Trademark) 7700 Sequence Detection System; ABI, and a fluorescence reagent SYBR Green; Molecular Probes] was carried out to compare the number of copies of the SREB2 gene contained in the chromosomes. As a result, the number of copies was 2 times or more in 7 offspring among 53 offspring, in comparison with that of the wild type, and these 7 offspring were identified as transgenic mice (F0). These transgenic mice (F0) were crossed with C57BL/6 mice, and the number of copies of the SREB2 gene in the chromosomes of the offspring (F1) was compared to analyze a germline transmission. As a result, the transmission of the incorporated gene to F1 was confirmed in 2 lines among 7 lines, and these 2 lines were designated YM2 and YM4. The number of the SREB2 genes incorporated into the chromosomes of YM2 and YM4 lines was approximately 10 copies and approximately 20 copies, respectively.

Example 3: Quantitative analysis of SREB2 mRNA

To confirm that the introduced polynucleotide actually functions to overexpress the SREB2 mRNA, the expression of the SREB2 mRNA in the brain of the transgenic mice was analyzed. From the F1 heterotransgenic mice (7-week-old) prepared in Example 2 and the littermate wild type mice, the forebrains and the cerebellums were taken and RNAs were isolated. Each RNA sample was digested with a DNase (DNase; Promega) to prevent the contamination of genomic DNAs. The number of copies of the SREB2 mRNA contained in each RNA sample was determined by the real-time PCR described in Example 2. A single-stranded cDNA synthesized from each RNA sample using a reverse transcriptase-polymerase chain reaction kit (Advantage RT-for-PCR kit; Clontech) was used as a template of the real-time PCR.

As a result of the real-time quantitative PCR, an overexpression of SREB2 mRNA approximately 4 times higher than that of the wild type was found in the forebrain of the F1 heterotransgenic mouse of the YM4 line. In this connection, an increased amount of SREB2 mRNA expressed in the cerebellum of the YM4 line was less than 2 times. In the F1 heterotransgenic mouse of the YM2 line, an overexpression of SREB2 mRNA approximately 2 times higher than that of the wild type was found in the forebrain, and an increased amount of SREB2 mRNA expressed in the cerebellum was less than 2 times.

Example 4: Morphological analysis of brain in SREB2-overexpressed transgenic mice

To analyze the effects of the SREB2 overexpression on the structure of the brain, the brains were taken from the YM2 and YM4 heterotransgenic mice and the littermate wild type mice. The weight of each brain was measured and the shape thereof observed.

The F1 heterotransgenic mice prepared in a similar fashion to that described in Example 2 was used to prepare F2 heterotransgenic mice of the YM2 and YM4 lines. In 5-week-old mice, it was found that the weight of the brain was lower than that of the wild type in the F2 heterotransgenic mice of the YM2 and YM4 lines (YM2: $0.36\pm0.01$ g, YM4: $0.33\pm0.01$ g, wild type: $0.46\pm0.01$ g, N=2 to 5). The decreasing rate of the brain weight in the YM4 line (28%) was higher than that in the YM2 line (22%), and this result correlated with the expression of SREB2. It was found from the morphological observation of sections of the brains that the cerebral ventricles were enlarged in accordance with a decreased size of the parenchyma of brain. The reduction of the parenchyma of brain and the enlargement of the cerebral ventricles accord with the phenotype observed in the brain of patients with schizophrenia.

These results indicate that morphological changes similar to those in the brain of patients with schizophrenia occur in the transgenic mice. Further, the similar phenotypes were observed in the two different lines, and this result indicates that these phenotypes were caused by the overexpression of SREB2.

Example 5: Behavioral analyses of SREB2-overexpressed transgenic mice

In the following behavioristic analyses, F3 heterotransgenic mice of the YM2 line, in which the decreasing rate of the brain weight was low, were used. A startle response analysis (prepulse inhibition test) is commonly-used as a quantitative analysis of an information processing activity which is disordered in patients with schizophrenia. When an analysis was carried out in accordance with the method described in "[3] Method of measuring schizophrenia-related disorders and method of detecting therapeutic effect on schizophrenia 1) Startle response analysis (prepulse inhibition test)", a reduction in prepulse inhibition (inhibition of a startle response by prepulse) was observed in the YM2 heterotransgenic mice. This result accords with the phenotype observed in patients with schizophrenia.

Further, 10 male transgenic mice (10-week-old or more) (Tg) and 10 male littermate wild type mice (WT) as a control group for comparison were used to carry out the following tests.

1) Prepulse inhibition test

When an analysis was carried out in accordance with the method described in "[3] Method of measuring schizophrenia-related disorders and method of detecting therapeutic effect on schizophrenia 1) Startle response analysis (prepulse inhibition test)", a significant reduction in prepulse inhibition (inhibition of a startle response by prepulse) was observed in the YM2 heterotransgenic mice (Tg) in comparison with the wild type (WT) as the control group (see Figure 2). In Figure 2, the abbreviation 3D shown in the horizontal axis indicates the result when a prepulse of 73 dB (20 ms) and a pulse of 120 dB (40 ms) were loaded. Similarly, the abbreviation 6D indicates the result when a prepulse of 76 dB (20 ms) and a pulse of 120 dB (40 ms) were loaded. The abbreviation 12D indicates the result when a prepulse of 82 dB (20 ms) and a pulse of 120 dB (40 ms) were loaded. The vertical axis indicates prepulse inhibition (%). This result showed that the SREB2-overexpressed transgenic mice exhibited disorders of an information processing activity. This phenotype accords with the phenotype observed in patients with schizophrenia.

2) Social behavior test

Disorders of social behavior are used as an index of negative symptoms of schizophrenia. When an analysis was carried out in accordance with the method described in "[3] Method of measuring schizophrenia-related disorders and method of detecting therapeutic effect on schizophrenia 2) Social behavior test", a significant reduction in the frequency of social investigation was observed in the YM2 heterotransgenic mice in comparison with the wild type as the control group (WT 66.8$\pm$4.2, Tg 54.2$\pm$3.9, $P<0.05$). This result showed that the SREB2-overexpressed transgenic mice exhibited disorders of social behavior. This phenotype accords with the phenotype observed in patients with schizophrenia.

3) Morris water maze test

In schizophrenia, cognitive impairment is caused in addition to positive symptoms and negative symptoms. The Morris water maze test is commonly used as a quantitative analysis of cognition. When an analysis was carried out in accordance with the method described in "[3] Method of measuring schizophrenia-related disorders and method of detecting therapeutic effect on schizophrenia 3) Morris water maze test", a significant extension in the goal latency to the platform during training trials was observed in the YM2 heterotransgenic mice (Tg) in comparison with the wild type (WT) as the control group (see Figure 3). In Figure 3, the horizontal axis indicates the number of training trials, and the vertical axis indicates the goal latency to the platform (seconds). Further, a significant reduction in the frequency of passing through the position in which the platform had been set (the fourth quadrant) during the probe trial was observed in the YM2 heterotransgenic mice (Tg) in comparison with the wild type (WT) as the control group (WT 7.3$\pm$0.9, Tg 3.1$\pm$0.9, $P<0.01$). These results showed that the SREB2-overexpressed transgenic mice exhibited cognitive impairment. This phenotype accords with the phenotype observed in patients with schizophrenia.
Further, a significant extension of the immobility time during swimming was observed in the YM2 heterotransgenic mice (Tg) in comparison with the wild type (WT) as the control group (see Figure 4). In Figure 4, the horizontal axis indicates the number of training trials, and the vertical axis indicates the immobility time (seconds). It was reported that an extension of the immobility time during swimming was involved in negative symptoms of schizophrenia (Noda et al. Br J Pharmacol. 116, 2531-2537, 1995). This result showed that the SREB2-overexpressed transgenic mice exhibited a phenotype similar to negative symptoms of schizophrenia.

4) Fear conditioning test

A fear conditioning test is known as a quantitative analysis for cognition. When an analysis was carried out in accordance with the method described in "[3] Method of measuring schizophrenia-related disorders and method of detecting therapeutic effect on schizophrenia 4) Fear conditioning test", a significant shortening of the immobility time was observed in the YM2 heterotransgenic mice in comparison with the wild type as the control group (percentage of immobility time per measuring time, WT 17.87$\pm$2.88, Tg 8.79$\pm$1.34, $P<0.05$). This result showed that the SREB2-overexpressed transgenic mice exhibited cognitive impairment. This phenotype accords with the phenotype observed in patients with schizophrenia.

The above results indicates that not only morphological changes in the brain but also behavioristic disorders similar to that in schizophrenia are caused in the SREB2-overexpressed transgenic mice YM2, and the transgenic mice can be used as a model animal of schizophrenia. It is possible to screen for a therapeutic agent for schizophrenia by using the SREB2 transgenic mice YM2 and analyzing the alleviation of schizophrenia-related disorders as an index.

Example 6: In vivo screening for therapeutic agent for schizophrenia, therapeutic agent for negative symptoms, and therapeutic agent for cognitive impairment using SREB2 transgenic mice

In vivo screening for a therapeutic agent is carried out in accordance with the startle response analysis, the social behavior test, the Morris water maze test, and the fear conditioning test, as described in Example 5. A test substance is suspended in a physiologic saline supplemented with 0.5% methylcellulose, and the suspension is intraperitoneally administered to mice. When a reduction in prepulse inhibition (inhibition of a startle response by prepulse), an alleviation of social behavior disorders, or an alleviation of memory and learning disorders is observed in a test-substance-administered SREB2 transgenic mouse group in comparison with a control group in which the physiologic saline supplemented with 0.5% methylcellulose is administered, it can be judged that the test substance has a therapeutic effect on schizophrenia, and the test substance can be selected as a therapeutic agent for schizophrenia.
When an alleviation of social behavior disorders is observed in the social behavior test, or an alleviation of immobility is observed in the Morris water maze test, it can be judged that the test substance has a therapeutic effect on negative symptoms of schizophrenia, and the test substance can be selected as a therapeutic agent for negative symptoms.
When an alleviation of memory and learning disorders is observed in the Morris water maze test and/or the fear conditioning test, it can be judged that the test substance has a therapeutic effect on cognitive impairment of schizophrenia, and the test substance can be selected as a therapeutic agent for cognitive impairment.

## INDUSTRIAL APPLICABILITY

According to the screening method of the present invention, substances useful as a therapeutic agent for schizophrenia, particularly a therapeutic agent for a negative symptom and/or cognitive impairment, can be screened for in vivo, and more effective therapeutic agents can be selected.
The transgenic animal of the present invention is useful as a model animal of schizophrenia that is a screening tool for a therapeutic agent for schizophrenia, particularly a therapeutic agent for a negative symptom and/or cognitive impairment. This screening tool can be used in screening for a therapeutic agent for schizophrenia.
A substance obtainable by the screening method of the present invention may be used as an active ingredient, together with a carrier, a filler, and/or other additives, to produce a pharmaceutical composition for treating schizophrenia, particularly a negative symptom and cognitive impairment.
Although the present invention has been described with reference to specific embodiments, various changes and modifications obvious to those skilled in the art are possible without departing from the scope of the appended claims.

## FREE TEXT IN SEQUENCE LISTING

The nucleotide sequence of SEQ ID NO: 1 in the sequence listing is a sequence for preparing a transgenic mouse. Each of the nucleotide sequences of SEQ ID NOS: 3-12 is an artificially synthesized primer sequence.

## Claims

1. A non-human transgenic animal exhibiting schizophrenic symptoms, wherein (1) a polynucleotide encoding a polypeptide comprising the amino acid sequence of SEQ ID NO: 2, and (2) a polynucleotide comprising a promoter are introduced.

2. The non-human transgenic animal according to claim 1, wherein the promoter is an exogenous promoter.

3. The non-human transgenic animal according to claim 1 or 2, wherein the polypeptide consists of the amino acid sequence of SEQ ID NO: 2.

4. The non-human transgenic animal according to any one of claims 1 to 3, wherein the promoter is a promoter capable of specifically expressing the polypeptide in the brain.

5. The non-human transgenic animal according to any one of claims 1 to 4, wherein the promoter is an $\alpha$-calcium-calmodulin-dependent kinase II promoter.

6. The non-human transgenic animal according to any one of claims 1 to 5, wherein the animal is a mouse.

7. The non-human transgenic animal according to any one of claims 1 to 6, wherein an amount of an mRNA expressed in the forebrain is 1.5 times or more, and less than 3 times in comparison with that in a wild type, the mRNA encoding a polypeptide comprising the amino acid sequence of SEQ ID NO: 2.

8. A model animal of schizophrenia, which is the non-human transgenic animal according to any one of claims 1 to 7.

9. The model animal of schizophrenia according to claim 8, which is a model animal of a negative symptom and/or cognitive impairment.

10. Use of the non-human transgenic animal according to any one of claims 1 to 7 in the detection of a therapeutic effect on schizophrenia.

11. The use according to claim 10, wherein the therapeutic effect on schizophrenia is a therapeutic effect on a negative symptom and/or cognitive impairment in schizophrenia.

12. A method of detecting a therapeutic effect of a test substance on schizophrenia, comprising the steps of:

administrating a test substance to the non-human transgenic animal according to any one of claims 1 to 7, and analyzing a schizophrenia-related disorder in the animal.

13. The method according to claim 12, wherein the analyzing step is a step of analyzing an alleviatory effect on a negative symptom and/or cognitive impairment in schizophrenia, and the therapeutic effect on schizophrenia is an alleviatory effect on a negative symptom and/or cognitive impairment.

14. Use of the non-human transgenic animal according to any one of claims 1 to 7 in the screening for a therapeutic agent for schizophrenia.

15. The use according to claim 14, wherein the therapeutic agent for schizophrenia is a therapeutic agent for a negative symptom and/or cognitive impairment.

16. A method of screening for a therapeutic agent for schizophrenia, comprising the steps of:

administrating a test substance to the non-human transgenic animal according to any one of claims 1 to 6, analyzing a schizophrenia-related disorder in the animal, and
selecting the substance exhibiting a therapeutic effect on schizophrenia.

17. The method according to claim 16, wherein the analyzing step is a step of analyzing an alleviatory effect on a negative symptom and/or cognitive impairment in schizophrenia, the selecting step is a step of selecting the substance exhibiting an alleviatory effect on a negative symptom and/or cognitive impairment in schizophrenia, and the therapeutic agent for schizophrenia is a therapeutic agent for a negative symptom and/or cognitive impairment.

18. A method of producing a pharmaceutical composition for treating schizophrenia, comprising the steps of:

screening a test substance by the method according to claim 16 or 17, and
preparing a medicament containing the substance obtained by the screening.

Figure 1

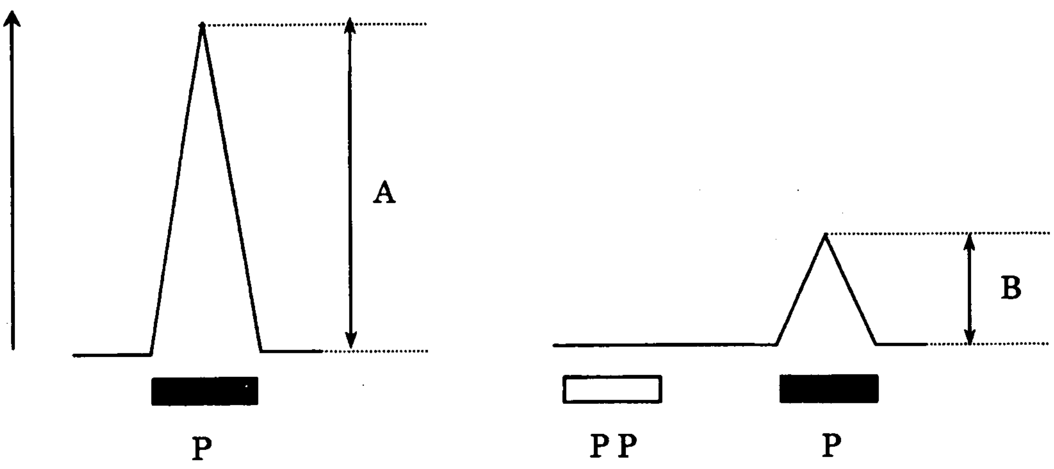


Figure 2

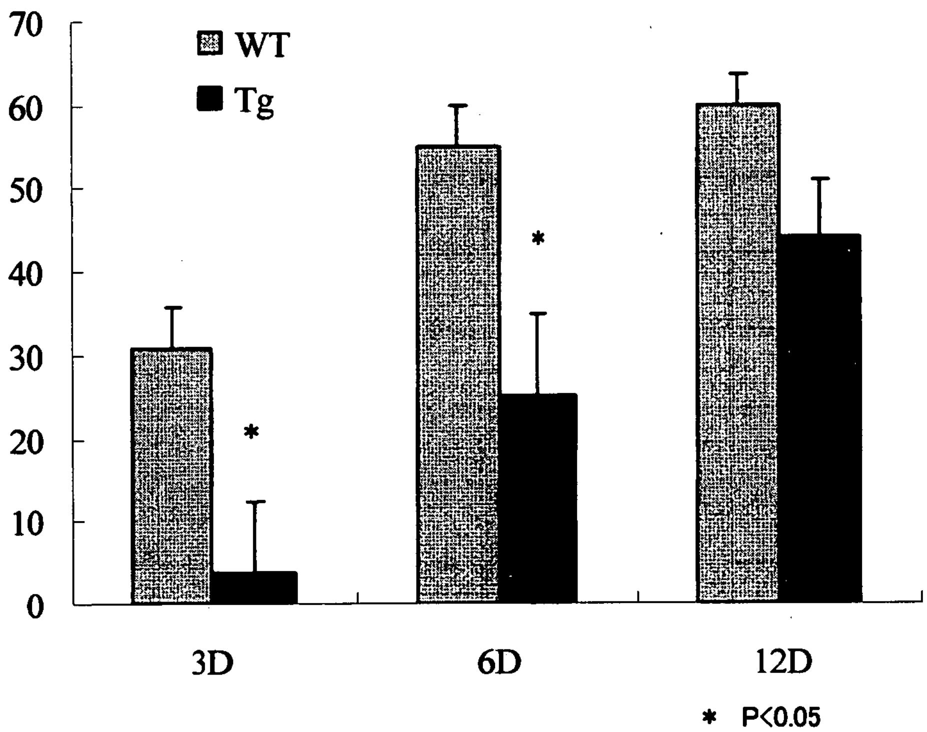

Figure 3

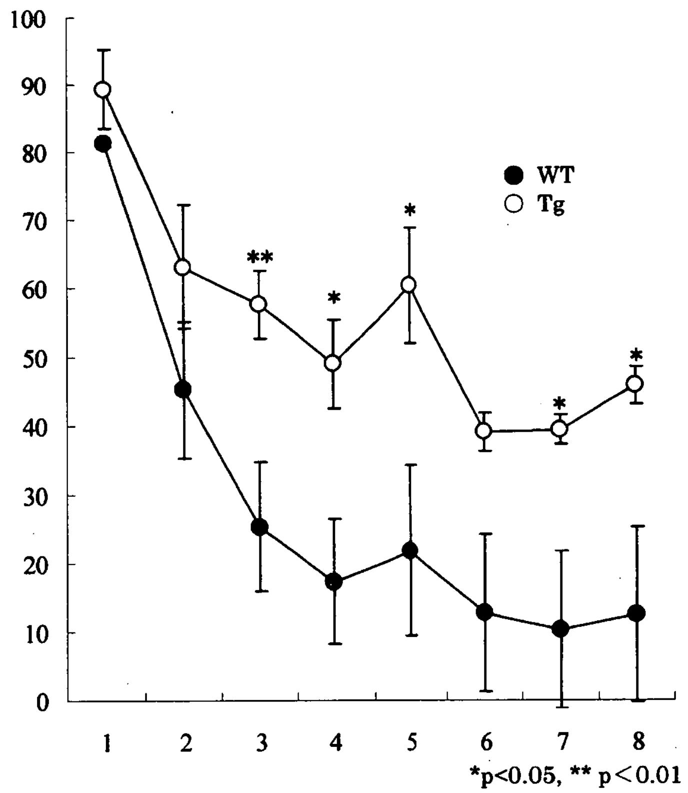


Figure 4

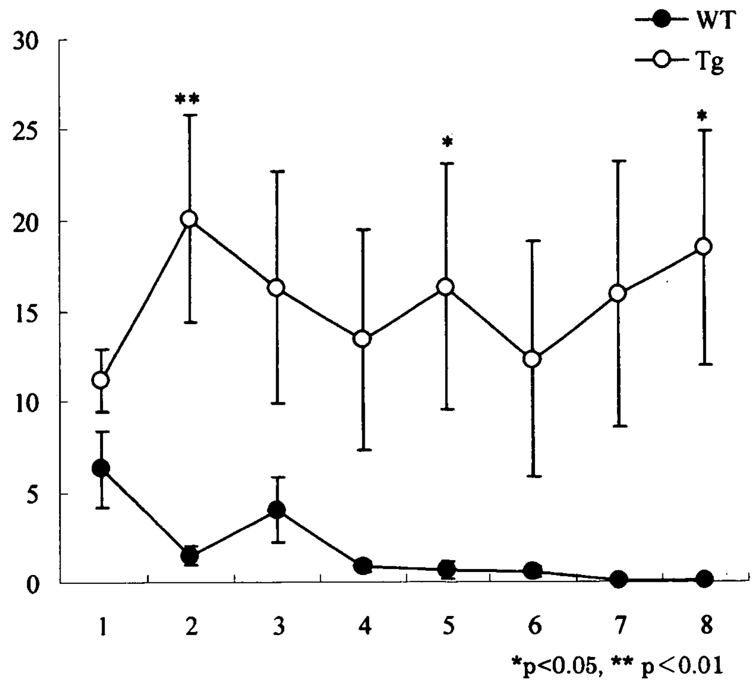

**INTERNATIONAL SEARCH REPORT**

International application No.
PCT/JP2006/320343

A. CLASSIFICATION OF SUBJECT MATTER
*A01K67/027*(2006.01)i, *A61K45/00*(2006.01)i, *A61P25/28*(2006.01)i, *C12N15/09*(2006.01)i, *G01N33/15*(2006.01)i, *G01N33/50*(2006.01)i

According to International Patent Classification (IPC) or to both national classification and IPC

B. FIELDS SEARCHED

Minimum documentation searched (classification system followed by classification symbols)
A01K67/027, A61K45/00, A61P25/28, C12N15/09, G01N33/15, G01N33/50

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS(STN), MEDLINE(STN), WPIDS(STN), JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X<br>Y | WO 1999/046378 A1 (Yamanouchi Pharmaceutical Co., Ltd.),<br>16 September, 1999 (16.09.99),<br>& AT 326531 T & AU 3276699 A<br>& DE 69931360 D1 & EP 1067183 A1<br>& EP 1067183 B1 & EP 1690939 A2<br>& US 6555344 B1 & US 6808899 B2<br>& US 2003/077662 A1 & US 2005/042683 A1 | 1-7<br>8-17 |
| X<br>Y | MATSUMOTO,M et al., An evolutionarily conserved G-protein coupled receptor family, SREB, expressed in the central nervous system., Biochem Biophys Res Commun. 2000 Jun 7; 272(2): 576-82. | 1-7<br>8-17 |

☒ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 November, 2006 (17.11.06) | 28 November, 2006 (28.11.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/320343

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO 2002/086147 A2 (PHARMACIA & UPJOHN CO.),<br>31 October, 2002 (31.10.02),<br>& CA 2441353 A1 & EP 1470245 A2<br>& JP 2004-528847 T & US 2003/224365 A1 | 1-7<br>8-17 |

Form PCT/ISA/210 (continuation of second sheet) (April 2005)

**INTERNATIONAL SEARCH REPORT**

International application No.
PCT/JP2006/320343

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 18
because they relate to parts of the international application that do not comply with the prescribed requirements to such an extent that no meaningful international search can be carried out, specifically:
A method for producing a pharmaceutical composition of claim 18 is a method for producing a pharmaceutical composition specified by the screening method of claim 16 or 17, however, there is no description of specific examples as such a pharmaceutical composition in the (continued to extra sheet)

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.
PCT/JP2006/320343

Continuation of Box No.II-2 of continuation of first sheet(2)

specification. Therefore, claim 18 lacks disclosure within the meaning of PCT Article 5 and support by the disclosure of the specification within the meaning of PCT Article 6.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9946378 A
- WO 02086147 A


### Non-patent literature cited in the description


- **LIEBERMAN et al.** *N Engl J Med.,* 2005, vol. 353, 1209-1223
- **MELISSA K. SPEAKING.** Schizophrenia. *National Institute of Mental Health,* August 2002, http://www.nimh.nih.gov/publicat/NIMHschizoph.pdf>
- *The New England Journal of Medicine, the U.S.A.,* 2005, vol. 353, 1209-1223
- *Behavioural Pharmacology, the United Kingdom,* 2000, vol. 11, 223-233
- *Biochemical and Biophysical Research Communications, the U.S.A.,* 2000, vol. 272, 576-582
- **MAYFORD, M. et al.** *Proc. Natl. Acad. Sci. USA,* 1990, vol. 93, 13250-13255
- **QUON, D. et al.** *Nature,* 1991, vol. 352, 239-241
- **VIDAL, M. et al.** *EMBO J,* 1990, vol. 9, 833-840
- **SAIKI, R. K. et al.** *Science,* 1988, vol. 239, 487-491
- *Animal Biotechnology,* 1990, vol. 1, 175-84
- **N.K.TERRETT ; M.GARDNER ; D.W.GORDON ; R.J.KOBYLECKI ; J.STEELE.** *Tetrahedron,* 1995, vol. 51, 8135-73
- **MANIATIS, T. et al.** Molecular Cloning- A Laboratory Manual. Cold Spring Harbor Laboratory, 1982
- **NODA et al.** *Br J Pharmacol.,* 1995, vol. 116, 2531-2537